# EUROPEAN PATENT SPECIFICATION

(11) **EP 1 827 514 B1**
(45) Date of publication and mention of the grant of the patent: **10.09.2008**
(21) Application number: 05825527.4
(22) Date of filing: 22.11.2005
(51) Int. Cl.: A61L 9/12

(54) **Device for selectively fragrancing and purifying air**
Vorrichtung zur selektiven Beduftung und Luftreinigung
Système de diffusion de fragrance sélective et de purification d'air

(30) Priority: 23.11.2004 US 630344 P
(43) Date of publication of application: 05.09.2007
(73) Proprietor: S.C. JOHNSON & SON, INC., Racine, Wisconsin 53403 (US)
(72) Inventor: CRAPSER, James, R., Racine, Wisconsin 53406 (US); GASPER, Thomas, P., Germantown, Wisconsin 53022 (US)
(74) Representative: Ruschke, Hans Edvard
(86) International application number: PCT/US2005/042548
(87) International publication number: WO 2006/058126

(56) References cited:
- EP-A- 0 546 214
- WO-A-03/011416
- US-A- 4 563 333
- US-A- 4 959 087
- US-A- 5 258 051
- US-A- 5 297 988
- US-A1- 2002 090 317
- US-B1- 6 325 475
- US-B1- 6 511 531

## Description

### Field of the Invention

The present invention relates to air freshening devices. In particular, this invention relates to air freshening devices that provide air purification in combination with fragrancing.

### Background of the Invention

The increase of outdoor air pollution over many years has created a greater awareness in people for the type of damage that outdoor air pollution can cause to one's health. What is not commonly known, however, is that indoor air pollution also exists and can have a significant affect on one's health. There have been recent Environmental Protection Agency studies that indicate that certain levels of air pollution indoors could be 2-5 times higher than the ambient outdoor air pollution level. It is estimated by some that, on rare occasions, these indoor air pollution levels can be 100 times higher than outdoor air pollution levels. This is an increasingly important matter that must be addressed, because some people spend 90% of their time indoors especially infants and the elderly. Additionally, some of these indoor pollutants present at these elevated levels could be contributing factors to frequent and unexplained headaches or sleepless nights that afflict numerous persons within the general population.

To combat or mitigate indoor air pollution, there have been numerous prior art apparatuses designed and manufactured for purifying air within enclosed, interior spaces. With more homes and offices becoming better insulated, an objective of air purifiers is to clear the indoor air of common pollutants, including dust, smoke, pollen, bacteria, soot, mold spores, animal dander, and other microscopic irritants, and thereby create a clean, healthy, fresh, and pleasant environment. Some of these apparatuses remove these contaminants from the air by generating ions by using complicated wire grid arrays or with high voltage electrode arrays to charge the contaminants for attraction to and removal by an oppositely charged surface. Some use fans for moving air and similar complicated apparatuses. Some of these prior art devices are mounted in large housings that contain fans and other complicated moving parts and filters. Often, the devices become clogged with pollutants, which requires disassembly of fan assemblies, replacement and/or repair of high generating voltage sources, extensive clearing of arrays of wires and electrodes that help create air movement, and replacement of filters that clog the apparatuses unless cleaned. These devices are certainly more complicated and perhaps more expensive than what some users may anticipate or desire.

Another aspect of air freshening separate from purifying or cleaning the air that is of interest to consumers is that of adding fragrance to the air. Odor modification is the intentional change of an odor by the addition of a more agreeable odor. Air fresheners are typical odor modifiers because they employ volatile fragrance agents for odor control by altering or masking a malodor to a more pleasant character or to an acceptable level. Air fresheners were initially used in bathrooms and kitchens and consequently, have tended to be more functional than attractive. Air fresheners are now used in bedrooms and living rooms, and consumers who wish to use air fresheners in these areas of the home may be reluctant to place an unattractive, functional container in these areas. What is needed is an air freshening device, such as a portable tabletop device, that provides air purification in combination with air fragrancing. Because a tabletop unit is typically near a chair or bed, one key consideration is the noise level of the device. It is desirable to provide to the user a quiet air freshening device that is not excessively noisy.

One prior art device that both filters and fragrances air is the AMCOR ClearAire Elite air purifier device, manufactured by The Amcor Group Ltd. (Long Island City, NY). This device uses aromatherapy beads as the fragrance supply for the device. However, aromatherapy beads have many disadvantages from both a manufacturing and sales standpoint and a consumer standpoint. From a manufacturing and sales standpoint, aromatherapy beads are very inexpensive to consumers, but consumers tend not to purchase refills regularly because the fragrance or scent generated by the beads lingers continuously, even when the beads have been completely consumed through the operation of the device. This is because aromatherapy beads emit a strong scent initially, but once the beads are consumed, the scent lingers continuously at a lower level, without the consumer realizing that the beads are already consumed. Thus, the consumer is misled into thinking that the device is still working. Consequently, the consumer is not getting effective fragrancing. Additionally, this prior art unit does not provide an option for providing multiple fragrances, nor does this prior art unit provide air or fragrance rate control or direction control. Furthermore, this prior art unit uses a filter that is suitable for filtering smoke, but not effective for filtering particles, such as dust, pollen, allergens, and the like.

Concerning the functions of air purifying and fragrancing, there are two classes of consumer needs regarding air purification and fragrancing: (1) allergy prone consumers; and (2) non-allergy prone consumers. Allergy prone consumers are more interested in the air purification capability of an air freshening device, because their need for the unit is to help them feel better. The allergy prone consumer will notice that such a device provides improved breathing, improved sleeping, or noticeably less dust in the room, all of which reinforces the idea of the air being cleaned. Allergy prone consumers are not likely to desire fragrancing and, therefore, providing an air freshening device with fragrance capability is of much less importance. By contrast, non-allergy prone consumers tend to focus less on the air purification affects of the air freshening device and are more likely to notice a general improvement in over air freshness, such as less dust in the room. Non-allergy prone consumers are, however, likely to desire fragrancing. What is needed is a common air purification device that is capable of meeting the needs of both the allergy prone and non-allergy prone consumers by providing air filtration and fragrancing capability that can be controlled.

Additionally, consumers in both classes use the sense of feel as a tactile indicator of how well the air freshening device is working. However, consumers may not want to feel air blowing on them directly. Thus, there is benefit in being able to control the direction of the clean and/or fragranced airflow. Today's air freshening devices typically blow air out of the front or top face of the unit, and the consumer has no directional control. As a result, the consumer has to adjust the position of the entire unit in order to change the direction of the air. What is needed is a way to adjust air direction of an air freshening device without adjusting the position of the entire unit.

Further, consumers generally don't want a "one size fits all" air freshening device. Consumers prefer an air freshening device that can be customized to meet their specific needs. Thus, it is beneficial to provide an air freshening device that is customizable to provide, for example, one fragrance vs. no fragrance, multiple fragrances vs. no fragrance, variable rate of fragranced air, directional control of fragranced air, variable rate of clean air only, and directional control of clean air only.

Consequently, it is desirable to develop an air purifying system, method, and device for employing the method that overcomes the limitations of prior art air freshening devices and simultaneously addresses a wide range of consumer needs. Specifically, the air purifying system should provide the capability for filtering a wide range of air pollutants, in conjunction with adding a fragrance to the air. The addition of fragrance to the air should also be able to be controlled by the consumer such that the fragrance function can be switched on or off as desired. Further, the system should allow the consumer to direct the airflow from the system when desired without having to move the entire system.

EP 0 546 214 describes a perfume dispenser with a plurality of individual compartments containing perfume arranged in an inner circle. A top plate has a grill consisting of a plurality of slots, which can be aligned with any one of the perfume compartments to enable perfume to be emitted from that compartment alone. In addition, air can be selectively injected into the side of the active compartment to release the perfume.

### Summary of the Invention

The invention is as defined in claim 1 below. In a primary embodiment an air purification device includes an air purification mechanism and an air fragrancing mechanism. The air purification mechanism of the device operates in a conventional manner to remove various contaminants present in the air flowing through the device through the use of a filter in order to provide a stream of purified or clean air at the outlet of the device. In addition, the device enables a user to selectively supply an amount of a fragrance to the air being cleaned by using the fragrancing mechanism of the device. The fragrance provided by the fragrance mechanism can be a single fragrance or one or more of a number of fragrances that are operably connected to the fragrance mechanism. The fragrance mechanism also enables an individual to control the particular amount of the one or more fragrances being dispensed by the fragrance mechanism, such that the user can vary the amount of fragrance dispensed from the device as necessary.

According to an embodiment the air purification device also includes an air outlet and directional member that enables the cleaned and/or fragranced air flowing through the outlet of the device to be directed when desired by an individual. By allowing the air to be directed where intended by the user of the device, the cleaned and/or fragranced airflow from the outlet of the device can be prevented from directly striking the user, or can be directed to an area where the flow of cleaned and fragranced air is desired.

The device may be supplied with an indicator capable of providing the user of the device with an indication that the fragrance supply utilized with the fragrance mechanism of the device has or is close to being depleted. The indicator enables the user to replace any depleted fragrance supplies in order to allow the fragrance mechanism to provide continuous fragrancing to the air passing through the device as desired by the user.

The device can be constructed in a manner which greatly reduces the noise produced by the device during operation. The reduction in noise allows the unit to be positioned in a wide variety of locations, such as a bedroom, where the operation of the device will not disturb the occupants of the particular location.

Numerous other aspects, features, and advantages of the present invention will be made apparent from the following detailed description taken together with the drawing figures.

### Brief Description of the Drawings

The drawings illustrate the best mode currently contemplated of practicing the present invention.

In the drawings:
Figure 1 is a schematic view of an air freshening device constructed according to the present invention;
Figure 2 is a perspective view of a first embodiment of the device of Figure 1;
Figure 3A is a side plan view of a fragrance dispensing mechanism incorporated in the device of Figure 1;
Figures 3B-3E are top plan views of varying operating positions of the fragrance dispensing mechanism of Figure 3A;
Figure 4A is a side schematic view of a second embodiment of the fragrance dispensing mechanism utilized in the device of Figure 1;
Figure 4B is a top plan view of the second embodiment of the fragrance dispensing mechanism of Figure 4A;
Figure 5 is a perspective view of a third embodiment of a fragrance dispensing mechanism utilized in the device of Figure 1;
Figures 6-9 are deleted.
Figure 10 is a schematic view of the second embodiment of the purification device constructed according to the present invention;
Figure 11A is a perspective view of a filter element utilized in the device of Figure 10;
Figure 11B is a cross-sectional view along line 11B-11B of Figure 11A;

### Detailed Description of the Invention

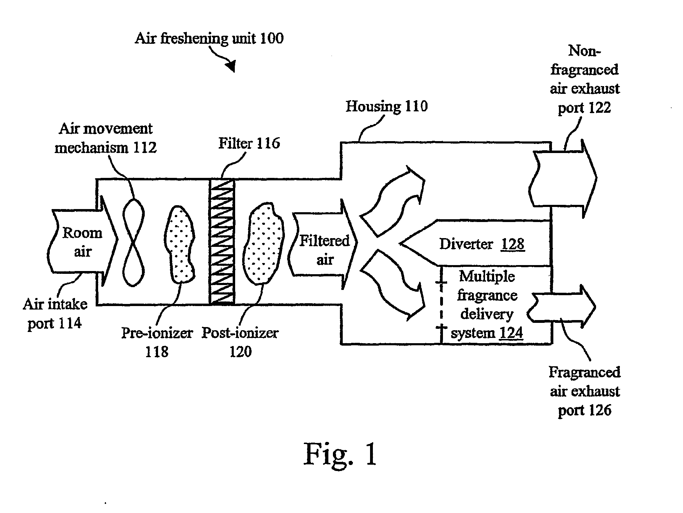

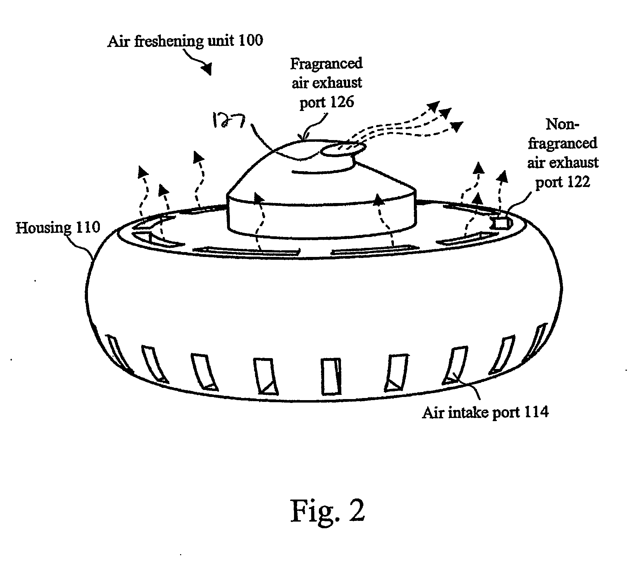

With reference now to the drawing figures in which like reference numerals designate parts throughout the disclosure, an air freshening unit that provides air purification in combination with fragrancing in accordance with a first embodiment of the invention is indicated generally at 100 in Figure 1. As shown in Figures 1 and 2, air freshening unit 100 includes a housing 110, within which is installed an air movement mechanism 112, such as a suitable fan, for drawing airflow into housing 110 via an air intake port 114. Airflow generated by air movement mechanism 112 pushes against and through a filter 116, which in a preferred embodiment is located on the exhaust side of air movement mechanism 112. In this same preferred embodiment, arranged between air movement mechanism 112 and filter 116 is a pre-ionizer 118, and arranged on the exhaust side of filter 116 is a post-ionizer 120. A first portion of the filtered air from the exhaust side of filter 116 exits housing 110 via a non-fragranced air exhaust port 122, and a second portion of the filtered air from the exhaust side of filter 116 passes through a multiple fragrance delivery system 124 and, subsequently, exits housing 110 via a fragranced air exhaust port 126. A diverter 128 forms the physical separation between the two airflow paths that lead to non-fragranced air exhaust port 122 and fragranced air exhaust port 126.

Housing 110 is representative of any lightweight plastic or metal enclosure for housing the elements of air freshening unit 100. Housing 110 is suitably sized and shaped for a tabletop air freshening device for home or office use. The footprint of housing 110 may be, for example, but is not limited to, rectangular, square, oval, or circular shape and of an area not more than, for example, 100 in². The height of housing 110 is, for example, 6 inches or less.

Air movement mechanism 112 is, for example, in a preferred embodiment, a standard, commercially available axially mounted multi-speed AC or DC electric fan for pushing air through filter 116 in the dirty fan configuration shown in Figure 1. Alternatively, the mechanism 112 can be positioned downstream of the filter 116 in a clean fan configuration to pull the air through the filter 116. For example, a squirrel cage fan (not shown) may be used for pulling, rather than pushing, air through filter 116. However, an axially mounted fan is preferred, because it creates higher head pressure against the filter 116, as compared to that of a squirrel cage fan. In either configuration, air movement mechanism 112 is capable of providing, for example, 30-100 cubic feet/minute (CFM) of airflow. Those skilled in the art will appreciate that the power supply (not shown) and electronic control (not shown) of a standard, multi-speed AC or DC electric fan is well known.

In a preferred embodiment, filter 116 is a conventional trapping filter that traps particulates. For example, filter 116 is a small footprint filter with a clean air delivery rate (CADR) rating of 80 or less, a pressure drop of less than 10-12 pascals, and an ozone emission of less than 0.05 ppm. CADR is an industry standard filter rating that is based on how much material is removed from the air in a single pass through a filter. Filter 116 includes a screen that is fine enough to filter out the desired particulates. The finer the particle, the finer the screen and, therefore, the greater the pressure needed to push air through the screen, which affects the possible CFM and the rate of air exchange in the room. In the case of air freshening unit 100, if, for example, air movement mechanism 112 provides approximately 90 CFM entering filter 116, approximately 55 CFM of airflow exits filter 116.

In an alternative embodiment, filter 116 is a nebulizer filtering system that forms a very fine spray or vapor. The vapor migrates through the air stream, by injecting the vapor across the incoming airflow, such as from vertically downwardly or upwardly, in a direction generally perpendicular to the stream flow. The fine particles of liquid wash the air free of particulate and the liquid is then filtered and re-nebulized, i.e., a supply and return system that uses a circulating pump (not shown).

Optionally, filter 116 may include an odor eliminating media, but at a sufficiently low level not to eliminate the fragrance that can be dispensed from the system 124 downstream of the filter 116. Filter 116 may include, for example, an anti-microbial coating, and may optionally include a charcoal prefilter.

Pre-ionizer 118 and post-ionizer 120 are each optional and serve as precipitating filter mechanisms used to enhance the function of filter 116. Pre-ionizer 118 and post-ionizer 120 are, in a preferred embodiment, standard, commercially available needle ionizers that use high voltage electricity to create negative electrons. These electrons run up the length of a pointed spike, or needle, where they stream into the air and attract oxygen molecules. At this point, they become negative ions that attach themselves to airborne particles. When enough negative ions attach to a particle, it gets too heavy to float in the air and drops to a surface in a process known as agglomeration, which effectively removes the particle from the circulating air. Those skilled in the art will appreciate that the power supply (not shown) and electronic control (not shown) for such a standard needle ionizer device is well known such that they need not be described in detail in this application.

Overall power for those components of the unit 100 that require power may be supplied via battery power or via a standard 110v or 220v household AC outlet.

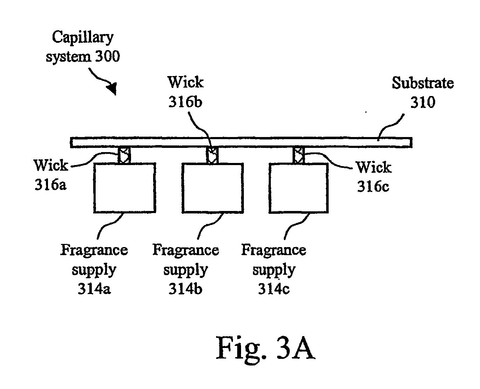

Air freshening unit 100 of the present invention generally provides fragrancing to the user by dispensing the fragrance into the exhaust stream of the device using multiple fragrance delivery system 124. Looking now at Figures 1 and 3A-5, multiple fragrance delivery system 124 is representative of a single or multi-fragrance delivery system that allows the user to turn on or off the selected fragrance. Multiple fragrance delivery system 124 includes, in a preferred embodiment, one or more supplies of fragrance oil and a capillary system for evaporating the oil into the airflow. Several embodiments and further details of multiple fragrance delivery system 124 are found in reference to Figures 3A, 4A, 4B, and 5.

Diverter 128 is representative of any well-known device, such as a baffle, for directing airflow along one or more airflow paths. In the case of air freshening unit 100, diverter 128 directs a portion of filtered air from the exhaust side of filter 116 toward non-fragranced air exhaust port 122 and also toward multiple fragrance delivery system 124, which supplies fragranced air exhaust port 126. The design of diverter 128 is such that in a preferred embodiment 0 to 10% of the filtered air-exiting filter 116 is directed into multiple fragrance delivery system 124 and, subsequently, exits fragranced air exhaust port 126. Consequently, 90 to 100% of the filtered air-exiting filter 116 is directed toward non-fragranced air exhaust port 122. However, the relative percentages of these air flows can be altered as desired, depending upon the particular use the unit 100 is designed for, such as by a movable baffle (not shown) capable of diverting between 0% and 100% of the filtered air through either exhaust port 122 or 126.

In a preferred embodiment, regardless of the CFM capability of air freshening unit 100, the design of diverter 128 limits the maximum airflow into multiple fragrance delivery system 124 and, subsequently, limits the exiting fragranced air exhaust. In this way, the maximum quantity of fragranced air delivered into the environment is controlled to an acceptable level (i.e., non-offensive level) and is not dependent on the overall CFM capability of air freshening unit 100.

The air delivery rate vs. noise specification of air freshening unit 100 is optimized to ensure a suitably high air delivery rate for achieving a preferred turn-over rate of four exchanges of air per hour in a 10x10 ft to 10x12 ft room, while at the same time maintaining a suitably low maximum noise specification, such as a noise specification not exceeding 50 decibels (dB). This optimization establishes an ideal performance on a "high setting" for a multi-speed fan as the air movement mechanism 112 that can move air through the filter 116 at approximately 55 CFM at a noise rating of <40 dB. Taking these specifications into account, in order to deliver an acceptable overall CADR rating for air freshening unit 100, the electric fan or other air movement mechanism 112 operates with a <100 CFM motor in combination with either filter 116 being a large footprint, high-pressure drop filter or, preferably, filter 116 being a small footprint, low-pressure drop (less than 12 pascals) filter. Additionally, in order for air freshening unit 100 to deliver an overall acceptable CADR rating, air movement mechanism 112 and filter 116 operate in combination with pre-ionizer 118 and post-ionizer 120, which enhance the function of filter 116 and thereby improve the overall CADR rating. With a multi-speed fan as the mechanism 112, the result is an air freshening unit 100 that provides a "low setting" specification of, for example, 30 CFM at 30 dB; a "medium setting" specification of, for example, 40 CFM at 35 dB; and a "high setting" specification of, for example, 50-55 CFM at 40 dB.

Looking now at Figures 3A-5, in operation, one or more supplies 314 of fragrance oil are installed within multiple fragrance delivery system 124 of air freshening unit 100. The user then selects a desired fragrance or no fragrance at all and activates air freshening unit 100, whereby air movement mechanism 112, pre-ionizer 118, and post-ionizer 120 are activated. In one example, air movement mechanism 112 is activated and, thus, draws air into air freshening unit 100 via air intake port 114. Air movement mechanism 112 pushes approximately 90 CFM of airflow into filter 116. Pre-ionizer 118 serves to remove particles from the airflow, as air passes from air movement mechanism 112 toward the intake of filter 116. Filter 116 then performs an additional filtering operation by trapping particulates that are not removed by the action of pre-ionizer 118. Approximately 55 CFM of filtered air exits the exhaust side of filter 116 and, subsequently, passes by post-ionizer 120, which removes additional particles remaining in the airflow, as a final air purification mechanism. As a result, filtered air is directed by diverter 128 toward non-fragranced air exhaust port 122 and multiple fragrance delivery system 124. The vast majority of airflow exits non-fragranced air exhaust port 122, and a much smaller controlled amount of airflow passes through multiple fragrance delivery system 124 and, subsequently, exits fragranced air exhaust port 126. In this example, if 55 CFM of filtered air exits the exhaust side of filter 116, no less than 90%, which is 49.5 CFM of airflow, is directed to non-fragranced air exhaust port 122 by diverter 128 and up to 10%, which is 5.5 CFM of airflow, passes through multiple fragrance delivery system 124 and, subsequently, exits fragranced air exhaust port 126.

Figure 2 illustrates air freshening unit 100 and shows an example housing 110 and an example fragranced air exhaust port 126 separate from the non-fragranced ports 122 that provide directional control of the fragranced air exiting through the port 108. In illustrated embodiments, housing 110 has a circular footprint that is suitably small to serve as a tabletop air purification and fragrancing unit for home or office use. All the elements of air freshening unit 100, as described in Figure 1, are mounted within or on housing 110. The air intake port 114 is implemented as multiple openings arranged around the lower perimeter of housing 110 for drawing room air from all directions (360 degrees) into the unit. The non-fragranced air exhaust ports 122 are implemented as multiple openings arranged around the upper perimeter of housing 110 for directing clean air in all directions (360 degrees) out of the unit 100. Further, fragranced air exhaust port 126 is implemented as a nozzle 127 that is swivelably mounted to the housing 110 by a suitable mechanism (not shown). The nozzle 127 is formed of a lightweight material, such as a molded plastic, and is installed at the top of housing 110. Due to the mechanism, the air exhaust port 126 and nozzle 127 may be rotated 360 degrees by the user in order to direct the fragranced air in any direction of his/her choosing.

Alternatively, a standard motion control system (not shown) can be provided in combination with this swivelably mounted air exhaust port 126 and nozzle 127 enabling the user electronic control on or remote from the unit 100 to select a specific stationary direction, a back and forth via a sweeping motion, or a continuous 360-degree rotational motion for the exhaust port 126 and nozzle 127.

Furthermore, other mechanisms (not shown) may be included within housing 110 to provide an oscillating air stream exiting non-fragranced air exhaust port 122. In one example, a rotating disk (not shown) with multiple openings arranged around its perimeter is mounted transversely within housing 110 in the clean air path and in close proximity to the multiple openings forming non-fragranced air exhaust port 122 that are arranged around the upper perimeter of housing 110. The rotating disk has fewer openings than those that form non-fragranced air exhaust port 122. The normal airflow-exiting filter 116 causes the disk to spin and, thus, the air will "walk around" air freshening unit 100 by using the rotating disk. The disk may include a stop-and-start lock mechanism (not shown) and a drag device (not shown) for controlling the speed of the rotation by friction.

Housing 110, air intake port 114, non-fragranced air exhaust port 122, and fragranced air exhaust port 126 are not limited to the example shown in Figure 2. Those skilled in the art will appreciate that housing 110 may be of any suitable alternatively shaped footprint. Additionally, air intake port 114, non-fragranced air exhaust port 122, and fragranced air exhaust port 126 may be implemented in any of several well-known mechanisms, such as moveable baffles (not shown). Furthermore, those skilled in the art will appreciate that standard control mechanisms (not shown), such as switches, dials, push buttons, or display devices, are mounted within or on housing 110 for user control of air freshening unit 100.

Looking now at Figures 3A-3E, a side view of a capillary system 300 for providing a selection of multiple fragrances is illustrated in accordance with a first embodiment of multiple fragrance delivery system 124 of air freshening unit 100. Capillary system 300 is formed as a wick-based system that incorporates a capillary member for delivering fragrance to the airflow. More specifically, capillary system 300 includes an elongated, thin, flat substrate 310 formed of, for example, molded plastic or glass. Arranged along the lower surface of substrate 310 are one or more capillary regions 312 associated with one or more fragrance supplies 314, respectively. Each fragrance supply 314 further includes a wick 316, which is positioned in direct contact with the lower surface of substrate 310.

Capillary regions 312 are representative of a wickable surface for enhancing vaporization of the fragrance oil into the air stream of air freshening unit 100. Capillary regions 312 are, in a preferred embodiment, 1 to 2 in² in area and are formed by one or more exposed capillary pathways (i.e., mechanical grooves) that are printed, etched, or molded into the surface of substrate 310. The critical characteristics of the capillary pathways may be optimized to accommodate the surface tension of specific fragrance formulations (e.g., scented oil). These characteristics include, for example, the angle of the groove walls, the sharpness of the lower corner, and a minimum depth specification, among others.

In a particularly preferred embodiment, capillary regions 312a-c are formed according to the principles and structure described in commonly-owned Patent Application No. 10/266,798 (the '798 application) entitled, "Wick-based delivery system with wick having small porosity sections," assigned to SC Johnson & Son, Inc., Racine, Wisconsin . The '798 application describes an evaporative device that includes a container for holding a liquid that has a porous wick extending through an opening, such that a portion of the wick contacts the liquid held within the container and a portion of the wick is exposed to the ambient environment, wherein the wick transfers the liquid from the container to the ambient air, and a portion of the wick is in communication with a surface of a capillary member. The surface of the capillary member has one or more exposed capillary pathways along which liquid, transferred by the wick from the container, is drawn by capillary action for dispersion to the ambient air.

An example of a wick-based fragrance supply suitable for use as fragrance supply 314a-c is a Glade^{®} brand Wisp^{™} fragrance oil refill bottle, manufactured by SC Johnson & Son, Inc. of Racine, Wisconsin. Example fragrances include cinnamon, apple, citrus, vanilla, floral fragrances, and tropical fruit fragrances. In the case where the capillary pathways of capillary regions 312a-c are optimized for use with the Glade^{®} brand Wisp^{™} fragrance oil, the groove walls have a specified angle, the lower corner has a specified angle, and the groove depth is at a specified minimum.

Wicks 316a, 316b, and 316c of fragrance supplies 314a, 314b, and 314c, respectively, are arranged linearly and in contact with the lower surface of substrate 310. A capillary region 312a is associated with wick 316a, a capillary region 312b is associated with wick 316b, and a capillary region 312c is associated with wick 316c. Only one wick 316a-c is in contact with and, therefore, engaged with, its associated capillary region 312a-c at a time. This is accomplished by the adjustment of the relative linear position of substrate 310 to fragrance supplies 314a, 314b, and 314c and wicks 316a, 316b, and 316c, either by holding fragrance supplies 314a, 314b, and 314c stationary and moving substrate 310 or by holding substrate 310 stationary and moving fragrance supplies 314a, 314b, and 314c. Alternatively, a standard motion control system (not shown) can be provided in combination with capillary system 300 and, thus, the user uses electronic control to select the desired fragrance by changing the position of the substrate 310.

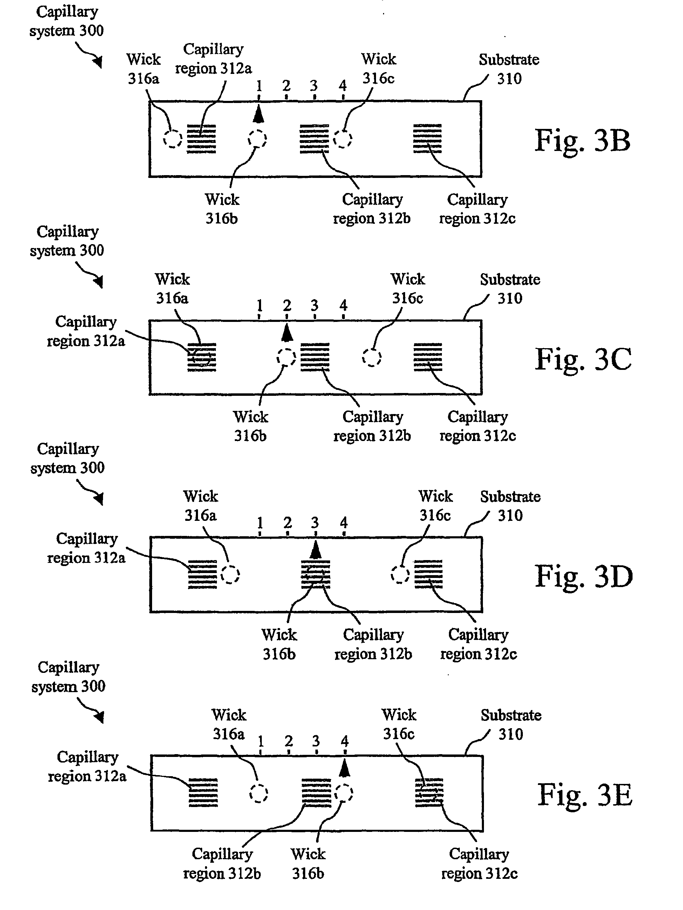

In a preferred embodiment, substrate 310 is slidably installed within housing 110 of air freshening unit 100 and aligned with and in contact with wicks 316a, 316b, and 316c of fragrance supplies 314a, 314b, and 314c, respectively, which are also installed at least partially within housing 110. Figure 3B illustrates a first position, wherein none of wicks 316a, 316b, or 316c is engaged with its associated capillary regions 312a, 312b or 312c, respectively, and, thus, no fragrance is selected, which thereby provides a means for the user to turn off fragrancing within air freshening unit 100. Figure 3C illustrates a second position, wherein wick 316a is engaged with capillary region 312a and wicks 316b and 316c are not engaged with capillary regions 312b and 312c, respectively, such that the fragrance of fragrance supply 314a is selected. Figure 3D illustrates a third position, wherein wick 316b is engaged with capillary region 312b and wicks 316a and 316c are not engaged with capillary regions 312a and 312c, respectively, such that the fragrance of fragrance supply 314b is selected. Figure 3E illustrates a fourth position, wherein wick 316c is engaged with capillary region 312c and wicks 316a and 316b are not engaged with capillary regions 312a and 312b, respectively, such that the fragrance of fragrance supply 314c is selected. This example fragrance selection is summarized in Table 1 below.

**Table 1 Fragrance selection positions of capillary system 300**

| **Position** | **Fragrance mode** |
|---|---|
| 1 | No fragrance selected |
| 2 | Fragrance #1 selected |
| 3 | Fragrance #2 selected |
| 4 | Fragrance #3 selected |

In operation, in the second, third, and fourth positions, as air flows across the surface of substrate 310 and, thus, across capillary regions 312a, 312b, and 312c, the liquid is transferred by wicks 316a, 316b, or 316c, respectively, from fragrance supplies 314a, 314b, or 314c, respectively, and drawn by the capillary action of capillary regions 312a, 312b or 312c, respectively, for dispersion by evaporation to the ambient air via fragranced air exhaust port 126 and nozzle 127.

In an alternative embodiment for the system 300, capillary regions 312a-c can be configured such that changing their position relative to wicks 316a-c provides contact with fewer or more capillary pathways in the associated regions 312a-c, which thereby provides a way to adjust the fragrance level as an alternative to simply adjusting the volume of airflow with air movement mechanism 112. This selective level of contact between the wicks 316a-c and the capillary regions 312a-c can be accomplished by any suitable means, such as by an additional mechanism (not shown) that moves the substrate 310 perpendicularly to the sliding direction shown in Figures 3B-3E, or simply by varying the configuration of the individual capillary regions 312a-c such as by forming wedge-shaped regions (not shown) on the substrate that provide contact with more area of the regions 312a-c as the substrate 310 is moved between positions 1, 2, 3, and 4 with respect to the wicks 316a-c. This example fragrance selection is summarized in Table 2 below.

**Table 2 Fragrance selection positions of capillary system 300 with varying capillary region 312a-c configurations**

| **Position** | **Fragrance mode** |
|---|---|
| 1 | No fragrance selected |
| 2 | Fragrance #1 - low level |
| 3 | Fragrance #1 - medium level |
| 4 | Fragrance #1 - high level |
| 5 | Fragrance #2 - low level |
| 6 | Fragrance #2 - medium level |
| 7 | Fragrance #2 - high level |
| 8 | Fragrance #3 - low level |
| 9 | Fragrance #3 - medium level |
| 10 | Fragrance #3 - high level |

In yet another alternative embodiment for the system 300, capillary regions 312a-c are designed to overlap in certain areas, so as to optionally provide a blend of fragrances from two or more wicks 316a-c. This example fragrance selection is summarized in Table 3 below.

**Table 3 Fragrance selection positions of capillary system 300 with fragrance blending**

| **Position** | **Fragrance mode** |
|---|---|
| 1 | No fragrance selected |
| 2 | Fragrance #1 selected |
| 3 | Blend of fragrance #1 and #2 selected |
| 4 | Fragrance #2 selected |
| 5 | Blend of fragrance #2 and #3 selected |
| 6 | Fragrance #3 selected |
| 7 | Blend of fragrance #3 and #1 selected |

Furthermore, multiple fragrance delivery system 124 is not limited to the above-mentioned example combinations. Those skilled in the art will appreciate that multiple fragrance delivery system 124 may be designed with a capillary system 300 that provides any number of combinations of fragrance levels and fragrance blends.

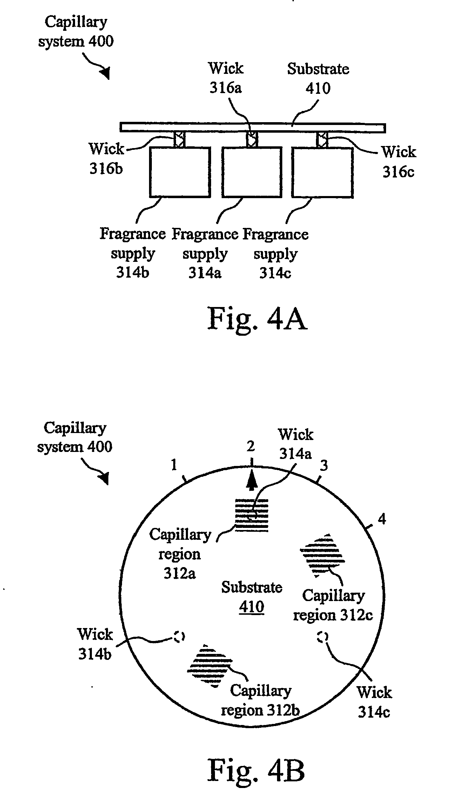

For example, referring now to Figures 4A-4B, a capillary system 400 is illustrated for providing a selection of multiple fragrances in accordance with a second embodiment of multiple fragrance delivery system 124 of air freshening unit 100. Capillary system 400 is a wick-based system that incorporates a capillary member for delivering fragrance to the airflow. In all respects, the system 400 is highly similar to capillary system 300 illustrated in Figures 3A-3E, except that elongated substrate 310 is replaced with a diskshaped, thin, flat substrate 410, likewise formed of, for example, molded plastic or glass. Arranged along the lower surface of substrate 410 are capillary regions 312a-c that are associated with fragrance supplies 314a-c and wicks 316a-c. Wicks 316a-c are positioned in direct contact with the lower surface of substrate 410. Because of the disk shape of substrate 410, fragrance supplies 314a-314c are arranged radially within the boundaries of the perimeter of substrate 410.

Similar to capillary system 300, in the capillary system 400, only one wick 316a-c is in contact with and, therefore, engaged with, its associated capillary region 312a-c at a time. This is accomplished by the user's adjusting the relative radial position of substrate 410 to fragrance supplies 314a-c and wicks 316a-c, either by holding fragrance supplies 314a-c stationary and rotating substrate 410 or by holding substrate 410 stationary and rotating fragrance supplies 314a-c. Substrate 410 may be designed similarly to the substrate 310 of capillary system 300 to achieve all combinations of fragrance levels and fragrance blends as shown in Tables 1, 2, and 3 and as previously described for the system 300.

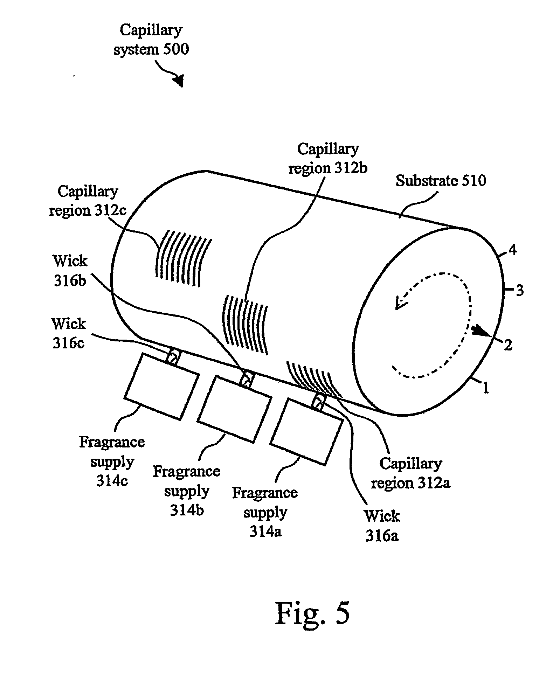

Looking now at Figure 5, a third embodiment of a capillary system 500 is illustrated for providing a selection of multiple fragrances in a multiple fragrance delivery system 124 of air freshening unit 100. Capillary system 500 is also formed as a wick-based system that incorporates a capillary member for delivering fragrance to the airflow similar to capillary systems 300 and 400 described previously, except that elongated substrate 310 is replaced with a cylindrical, thin, substrate 510, formed of, for example, molded plastic or glass. Arranged along the outer surface of substrate 510 are capillary regions 312 that are associated with fragrance supplies 314a-c and wicks 316a-c. Wicks 316a-c are positioned in direct contact with the outer surface of substrate 510. Fragrance supplies 314a-c are arranged linearly along the length of substrate 510.

As with capillary system 300, only one wick 316a-c is in contact with and, therefore, engaged with, its associated capillary region 312a-c at a time. This is accomplished by the user's adjusting the relative circular position of substrate 510 to fragrance supplies 314a-c and wicks 316a-c, either by holding fragrance supplies 314a-c stationary and rotating substrate 510 or by holding substrate 510 stationary and rotating fragrance supplies 314a-c. Substrate 510 may be designed to achieve all combinations of fragrance levels and fragrance blends as shown in Tables 1, 2, and 3 and as described previously for the system 300.

With reference to Figures 1 through 5 and all embodiments of multiple fragrance delivery system 124 within air freshening unit 100, such as capillary system 300, capillary system 400, and capillary system 500 , the selection of one of the multiple fragrances or no fragrance at all is performed via manual manipulation of the elements of multiple fragrance delivery system 124 by the user or, alternatively, a standard motion control system (not shown) is provided within air freshening unit 100 and, thus, the user uses electronic control to select the desired mode. The inclusion of a motion control system within air freshening unit 100 also allows the unit to be timer controlled. For example, air freshening unit 100 includes well-known electronics (not shown) that allows the user to select when air freshening unit 100 is automatically turned on or off and also to automatically select a given fragrance at a given time of day for a given amount of time, all under automatic control.

Furthermore, with continuing reference to Figures 1 through 5 and all embodiments of multiple fragrance delivery system 124 within air freshening unit 100, such as capillary system 300, capillary system 400, and capillary system 500, the physical assembly forming multiple fragrance delivery system 124 or the supplies 314 are easily removable from air freshening unit 100, such that the user can easily and conveniently replace the fragrance supply i.e., fragrance supplies 314 or aroma beads, when depleted.

In yet another embodiment, multiple fragrance delivery system 124 of air freshening unit 100 can be formed with one or more fragranced air pulsing devices, such as, one or more devices that create a puff of fragrance at time intervals by using a transducer unit (not shown) rather than by using the airflow created by air movement mechanism 112. Such a fragrance puffing mechanism is found, for example, in the Glade^{®} brand Wisp^{™} home fragrancer device manufactured by SC Johnson & Son, Inc. of Racine, Wisconsin, which uses a vibrating orifice plate-type atomizing dispenser in combination with a fragrance supply reservoir as disclosed in commonly-owned U.S. Patent Nos. 6,446,880, 6,293,474, 6,341,732, and 6,450,419, assigned to S.C. Johnson & Son, Inc.

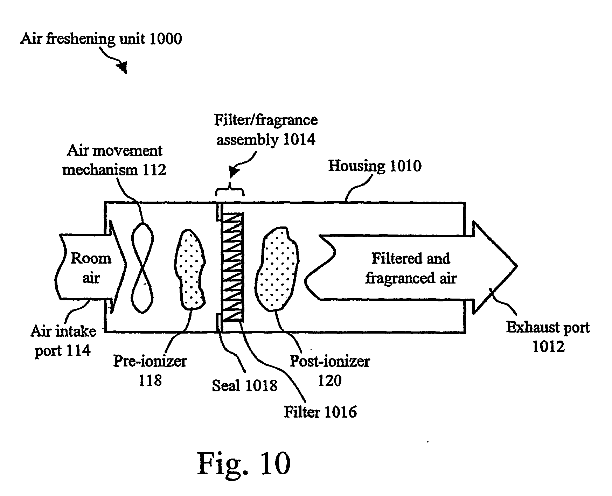

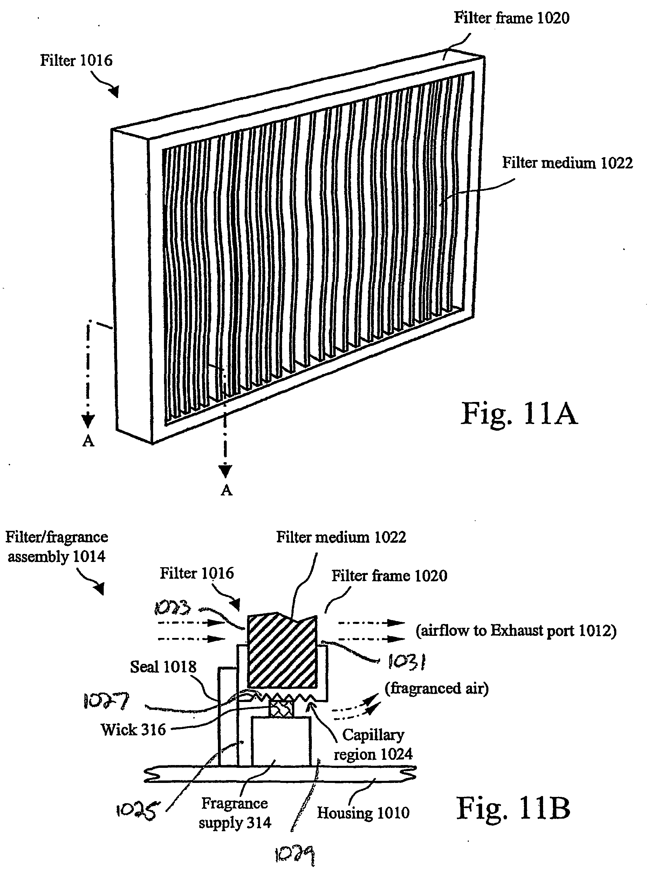

Looking now at Figures 10-11B, a functional diagram of an air freshening unit 1000 is illustrated for providing air purification in combination with fragrancing in accordance with a second embodiment of the invention where air freshening unit 1000 integrates the fragrancing mechanism within the filtration mechanism of the unit 1000. Thus, air freshening unit 1000 differs from air freshening unit 100 in that it does not provide a clean air only mode.

Air freshening unit 1000 includes a housing 1010, within which is installed air movement mechanism 112 for drawing airflow into housing 1010 via air intake port 114. Airflow generated by air movement mechanism 112 pushes against and through a filter/fragrance assembly 1014 that is formed of a filter 1016 abutted against a seal 1018 that sealingly engages the housing 1010 along its outer periphery and the filter 1016 along its inner periphery, to ensure airflow only through the filter 1016. Filter/fragrance assembly 1014 is preferably located on the exhaust side of air movement mechanism 112 but can also be disposed upstream of the mechanism 112. Arranged between air movement mechanism 112 and filter/fragrance assembly 1014 is pre-ionizer 118, and arranged on the exhaust side of filter/fragrance assembly 1014 is post-ionizer 120. The filtered/fragranced air from the exhaust side of filter/fragrance assembly 1014 exits housing 1100 via an exhaust port 1012. Air movement mechanism 112, air intake port 114, pre-ionizer 118, and post-ionizer 120 are formed similarly to the corresponding components illustrated and describe with regard to Figure 1.

Housing 1010 is representative of any lightweight plastic or metal enclosure for housing the elements of air freshening unit 1000. Housing 1010 is suitably sized and shaped for a tabletop air freshening device for home or office use. The footprint of housing 1010 may be, for example, but not limited to, rectangular, square, oval, or circular shape and of an area not more than, for example, 100 in². This height of housing 1010 is, in a preferred embodiment, six (6) inches or less.

Looking now at Figures 11A-11B, filter 1016 is formed with a frame 1020 positioned around a filter medium 1022 and installed within air freshening unit 1000. The intake side 1023 of filter frame 1020 abuts seal 1018, which thereby forces the full airflow from air movement mechanism 112 to pass through filter medium 1022 of filter 1016. A capillary region 1024 is formed in an outer surface of filter frame 1020, and wick 316 of one fragrance supply 314 is in direct contact with capillary region 1024. In a preferred embodiment, and as described previously, capillary region 1024 is formed according to the principles described in the '798 application.

Fragrance supply 314 is located in a cavity 1025 bounded by seal 1018 and housing 1010. Filter 1016, with its filter frame 1020, is designed specifically to function in combination with a specific brand of fragrance supply 314, such as, for example, the Glade^{®} brand Wisp^{™} fragrance oil refill bottle, manufactured by SC Johnson & Son, Inc. of Racine, Wisconsin. Filter 1016 is optimized for a specific fragrance to maximize the effectiveness of the unit 1000.

In operation, fragrance supply 314 sits in cavity or open well 1025 in close proximity to filter frame 1020, with its wick 316 in contact with capillary region 1024. The surface of capillary region 1024 has one or more exposed capillary pathways 1027 along which liquid, transferred by wick 316 from fragrance supply 314, is drawn by capillary action for dispersion to the ambient air. Air movement mechanism 112 is activated and, because fragrance supply 314 is mounted within the cavity 1025, a low-pressure area is formed by the velocity of the air stream passing by the cavity opening 1029, i.e., caused by the Venturi effect. The fragrance vaporizes within capillary region 1024 and is pulled out of the cavity opening 1029 and into the main airflow on the exhaust side 1031 of filter 1016 and, subsequently, mixes with the filtered air and is directed toward exhaust port 1012, as the air passes through filter 1016. Fragrance is delivered to the environment along with clean air until the supply of fragrance oil within fragrance supply 314 is depleted.

In an alternative embodiment, a hole (not shown) coupled to capillary region 1024 passes through filter frame 1020, so that filter medium 1022 is fragranced directly through the hole from the fragrance contained within capillary region 1024. In another alternative embodiment, filter frame 1020 includes an extending edge (not shown) like a windshield wiper blade along the exhaust side of filter 1016, and capillary region 1024 is coupled to this edge for the fragrance dispensing the fragrance from the edge into the passing air stream.

Like air freshening unit 100, the air delivery rate vs. noise specification of air freshening unit 1000 is optimized to ensure a suitably high air delivery rate for achieving a preferred turn-over rate of four exchanges of air per hour in a 10x10 ft to 10x12 ft room, while at the same time maintaining a suitably low maximum noise specification, such as a noise specification not exceeding 50 decibels (dB). This optimization establishes an ideal performance on a "high setting" for a multi-speed fan used as the air movement mechanism 112 to move the air through the filter 1016 at approximately 55 CFM at a noise rating of <40 dB. Taking these specifications into account, in order to deliver an acceptable overall CADR rating for air freshening unit 1000, the electric fan or other air movement mechanism 112 operates with a <100 CFM motor, in combination with either filter 1016 or filter 1200 being a large footprint, high-pressure drop filter or, preferably, filter 1016 or filter 1200 being a small footprint, low-pressure drop (less than 12 pascals) filter. Additionally, in order for air freshening unit 1000 to deliver an overall acceptable CADR rating, air movement mechanism 112 and filter/fragrance assembly 1014 are operating in combination with pre-ionizer 118 and post-ionizer 120, which enhance the function of filter/fragrance assembly 1014 and thereby improve the overall CADR rating. With a multi-speed fan in the mechanism 112, the result is an air freshening unit 1000 that provides a "low setting" specification of, for example, 30 CFM at 30 dB, a "medium setting" specification of, for example, 40 CFM at 35 dB, and a "high setting" specification of, for example, 50-55 CFM at 40 dB.

With reference to Figures 11 , air freshening unit 1000 may, alternatively, include well-known electronics (not shown) that allow the user to select when air freshening unit 1000 is automatically turned on or off or to change speeds via a timing mechanism.

Additionally, and with reference to Figures 11 , in an alternative embodiment the fragrance delivery mechanism may be integrated as part of the fan mechanism, such as in the impeller (not shown), of air movement mechanism 112. For example, a fragrance oil reservoir (not shown) and capillaries (not shown) are molded into the fan blade (not shown) itself. Such a design requires replacing the fan blade when the fragrance is depleted or when a different fragrance is desired. Another way to transport the fragrance oil from a refill supply to the fan blade is via a capillary system (not shown) that wicks the fragrance onto the fan blade by the wick contacting the spindle of the fan blade and the oil migrating into the fan blade due to the capillary and spinning action and, thus, the fan blade carries the fragrance as it spins.

With reference to air freshening unlit 100 and to air freshening unit 1000, various indicator mechanisms or "use-up queues" may also be included to let the user know whether, for example, the fragrance supply 314 or 1220 is depleted and, thus, needs replacing or to let the user know whether, for example, the CFM of the unit is reduced and, thus, the filter 116, 1016, or 1200 needs cleaning or replacing.

In a first embodiment for a use-up queue, one or more sight windows (not shown) formed of any transparent material, such as plastic or glass, are provided within housing 110 or the assembly of multiple fragrance delivery system 124. The sight windows allow the user to visually inspect the fluid level of the fragrance reservoir of each fragrance supply 314.

In a second embodiment for the use-up queue, one or more light sources (not shown), light detection devices (not shown), and electronic indicators (not shown) are provided with each fragrance supply 314. In this way, the intensity of light passing through the fragrance reservoir of each fragrance supply 314 is measured. An increase in light intensity indicates that the fragrance reservoir is empty, as the light is not passing through the fragrance oil, but instead, is passing through air only. For example, a light source, such as room light or a light-emitting diode (LED), may be piped via a fiber optic to pass through the lower region of the transparent fragrance reservoir of each fragrance supply 314. The light is directed toward a light detection device, such as a standard photocell that is located on the opposite side of the transparent fragrance reservoir. When fragrance liquid is present within the fragrance reservoir, a certain light intensity is expected; however, when the liquid is depleted, the photocell detects an increase in light intensity, which produces an electronic flag to the user that the fragrance reservoir is empty or nearly empty and, thus, activates a "low level" indicator to the user, such as an LED mounted upon housing 110 or 1010.

In a third embodiment for a use-up queue, a standard airflow sensor (not shown) is positioned in the airflow path of non-fragranced air exhaust port 122 and fragranced air exhaust port 126 of air freshening unit 100 and in the airflow path of exhaust port 1012 of air freshening unit 1000. When the airflow falls below a certain predetermined CFM, the airflow sensor activates an electronic flag (not shown), such as an LED mounted upon housing 110 or 1010, to the user that the filter requires cleaning or replacement.

Further, those skilled in the art will appreciate that other well-known manual, mechanical, or electronic use-up queue mechanisms are possible for use within the units 100 and/or 1000 in addition to those described above.

## Claims

1. An air cleaning device (100) comprising:
a) a housing (110);
b) an air purification mechanism (116) disposed within the housing and operable to remove contaminants within an airflow passing through the housing; and
c) a fragrancing mechanism (124) disposed within the housing and operable to dispense an amount of a fragrance into the airflow passing through the housing, wherein the fragrancing mechanism (124) is selectively operable and comprises:
a) at least one fragrance supply (314a, - 314c) comprising a wick; and
b) a fragrance dispenser (310) selectively connectable to the at least one fragrance supply (314a - 314c);
**characterized in that** the fragrance dispenser includes a substrate having at least one region (312a-312c) with capillary pathways that are printed, etched or molded on its surface wherein said at least one region can be selectively positioned in contact with the at least one fragrance supply (314a-314c); whereby when the wick is in contact with the capillary region, fragrance is drawn by capillary action from the fragrance supply (314a, 314c) and dispensed to the airflow.

2. The device of claim 1 further comprising an airflow diverter (128) disposed within the housing and operable to divert at least a portion of the airflow passing through the housing through the fragrancing mechanism (124).

3. The device of claim 2 further comprising:
a) a fragrance airflow outlet (126) located downstream of the fragrancing mechanism (124); and
b) a moveable nozzle (127) operably connected to the fragrance airflow outlet (126).

4. The device of claim 1 wherein the fragrance dispenser (310) is moveable with respect to the at least one fragrance supply.

5. The device of claim 1 wherein the at least one capillary region (312a - 312c) is incrementally engageable with the at least one fragrance supply.

6. The device of claim 1 wherein the at least one fragrance supply (314a-314c) is moveable with respect to the fragrance dispenser (310).

7. The device of claim 1 wherein the fragrancing mechanism (124) comprises:
a) a first fragrance supply (314a);
b) a second fragrance supply (314b); and
c) a fragrance dispenser (310) selectively connectable to the first supply (314a), the second supply (314b), or neither of the first supply or the second supply.

8. The device of claim 7 wherein the fragrance dispenser (310) is also operably connectable to both of the first supply (314a) and the second supply (314b).

9. The device of claim 1 wherein the air purification mechanism (116) and the fragrancing mechanism (124) are formed in a single structure (1014) disposed in the housing (1010).

10. The device of claim 9 wherein the single structure (1014) comprises:
a) a filter medium frame (1020) adapted to receive and retain a filter medium (1022) therein; and
b) a fragrance dispensing mechanism (314, 316) disposed within the frame (1020).

11. The device of claim 10 wherein the fragrance dispensing mechanism includes at least one capillary region (1024) that is engageable with at least one fragrance supply (314).

12. The device of claim 10 wherein the fragrance dispensing mechanism comprises:
a) a fragrance reservoir (314) disposed within the frame (1020) and adapted to hold an mount of a fragrance composition therein; and
b) a plurality of dispensing openings (1027) in fluid communication with the reservoir (314) and with an exterior surface of the frame (1020) to enable the fragrance composition to be dispensed from the openings (1027) into the airflow.

## Patentansprüche

1. Luftreinigungsvorrichtung (100) mit:
(a) einem Gehäuse (110);
(b) einer im Gehäuse angeordneten Luftreinigungsmechanik (116), mit der sich Verunreinigungen aus einer durch das Gehäuse hindurch tretenden Luftströmung entfernen lassen; und
(c) einer im Gehäuse angeordneten Beduftungsmechanik (124), mit der eine Menge eines Duftstoffs in die durch das Gehäuse hindurch tretende Luftströmung freisetzbar ist, wobei die Beduftungsmechanik (124) wahlweise betätigbar ist und
(a) mindestens einen Duftstoffvorrat (314a-314c) und einen Docht sowie
(b) eine Duftstoff-Freisetzeinrichtung (310) aufweist, die wahlweise mit dem mindestens einen Duftstoffvorrat (314a-314c) verbindbar ist;
**dadurch gekennzeichnet, dass** die Duftstoff-Freisetzeinrichtung ein Substrat mit mindestens einem Bereich (312a-312c) aufweist, in dessen Oberfläche durch Drucken, Ätzen oder Formgebung kapillare Kanäle ausgebildet sind, wobei der mindestens eine Bereich wahlweise in Berührung mit dem mindestens einen Duftstoffvorrat (314a-314c) bringbar ist und bei mit dem Kapillarbereich in Berührung stehendem Docht Duftstoff kapillar aus dem Duftstoffvorrat (314a-314c) gezogen und in die Luftströmung freigesetzt wird.

2. Vorrichtung nach Anspruch 1, weiterhin mit einer im Gehäuse angeordneten Luftstrom-Umlenkeinrichtung (128), die mindestens einen Teil der Luftströmung im Gehäuse in die Beduftungsmechanik (124) umlenken kann.

3. Vorrichtung nach Anspruch 2, weiterhin mit:
(a) einem Auslass (126) für die beduftete Luftströmung stromabwärts der Beduftungsmechanik (124) und
(b) einer mit dem Auslass (126) verbundenen bewegbaren Düse (127).

4. Vorrichtung nach Anspruch 1, bei der die Duftstoff-Freisetzeinrichtung (310) relativ zu dem mindestens einen Duftstoffvorrat bewegbar ist.

5. Vorrichtung nach Anspruch 1, bei der der mindestens eine Kapillarbereich (413a-314c) relativ zur Duftstoff-Freisetzeinrichtung (310) bewegbar ist.

6. Vorrichtung nach Anspruch 1, bei der der mindestens eine Duftstoffvorrat (314a-314c) relativ zur Duftstoff-Freisetzeinrichtung (310) bewegbar ist.

7. Vorrichtung nach Anspruch 1, deren Beduftungsmechanik (124) aufweist:
(a) einen ersten Duftstoffvorrat (314a),
(b) einen zweiten Duftstoffvorrat (314b) und
(c) eine Duftstoff-Freisetzeinrichtung (310), die wahlweise mit dem ersten oder dem zweiten Vorrat (314a bzw. 314b) oder keinem von beiden verbindbar ist.

8. Vorrichtung nach Anspruch 7, bei der die Duftstoff-Freisetzeinrichtung (310) auch mit sowohl dem ersten als auch dem zweiten Vorrat (314a, 314b) verbindbar ist.

9. Vorrichtung nach Anspruch 1, bei der die Luftreinigungs- und die Beduftungsmechanik (116, 124) als eine einzige Struktur (1014) im Gehäuse (1010) ausgebildet sind.

10. Vorrichtung nach Anspruch 9, bei der die einzige Struktur (1014) aufweist:
(a) einen Filterrahmen (1020), der ein Filtermittel (1022) aufnehmen und haltern kann, und
(b) eine im Rahmen (1020) angeordnete Duftstoff-Freisetzmechanik (314, 316).

11. Vorrichtung nach Anspruch 10, deren Duftstoff-Freisetzmechanik mindestens einen Kapillarbereich (1024) aufweist, der mit dem mindestens einen Duftstoffvorrat (314) in Berührung bringbar ist.

12. Vorrichtung nach Anspruch 10, deren Duftstoff-Freisetzmechanik aufweist:
(a) ein Duftstoff-Reservoir (314) im Rahmen (1020), das eine Menge einer Duftstoff-Zusammensetzung aufnehmen kann, und
(b) eine Vielzahl von Ausgabeöffnungen (1027), die mit dem Reservoir (314) und einer Außenfläche des Rahmens (1020) in Strömungsverbindung stehen und durch die die Duftstoff-Zusammensetzung in die Luftströmung freisetzbar ist.

## Revendications

1. Dispositif de nettoyage d'air (100) comprenant :
a) un boîtier (110) ;
b) un mécanisme de purification d'air (116) disposé à l'intérieur du boîtier et capable de fonctionner pour retirer les contaminants à l'intérieur d'un flux d'air passant à travers le boîtier ; et
c) un mécanisme de production de fragrance (124) disposé à l'intérieur du boîtier et capable de fonctionner pour distribuer une quantité de fragrance à l'intérieur du flux d'air passant à travers le boîtier, dans lequel le mécanisme de production de fragrance (124) est capable de fonctionner sélectivement et comprend :
a) au moins une amenée de fragrance (314a, 314c) comprenant une mèche ; et
b) un distributeur de fragrance (310) pouvant être raccordé sélectivement à la au moins une amenée de fragrance (314a-314c) ;
**caractérisé en ce que** le distributeur de fragrance comporte un substrat présentant au moins une zone (312a-312c) dotée de voies d'accès capillaires qui sont imprimées, gravées ou moulées sur sa surface dans lequel ladite au moins une zone peut être positionnée sélectivement en contact avec la au moins une amenée de fragrance (314a-314c) ; sachant que lorsque la mèche est en contact avec la zone capillaire, la fragrance est attirée par l'action capillaire depuis l'amenée de fragrance (314a, 314c) et distribuée dans le flux d'air.

2. Dispositif selon la revendication 1, comprenant en outre un déflecteur de flux d'air (128) disposé à l'intérieur du boîtier et capable de fonctionner pour dévier au moins une partie du flux d'air passant à travers le boîtier à travers le mécanisme de production de fragrance (124).

3. Dispositif selon la revendication 2, comprenant en outre :
a) une sortie de flux d'air de fragrance (126) positionnée en aval du mécanisme de production de fragrance (124) ; et
b) une buse mobile (127) raccordée de manière opérationnelle à la sortie de flux d'air de fragrance (126).

4. Dispositif selon la revendication 2, dans lequel le distributeur de fragrance (310) est mobile par rapport à la au moins une amenée de fragrance.

5. Dispositif selon la revendication 1, dans lequel la au moins une zone capillaire (312a-312c) peut être mise en prise de manière incrémentielle avec la au moins une amenée de fragrance.

6. Dispositif selon la revendication 1, dans lequel la au moins une amenée de fragrance (314a-314c) est mobile par rapport au distributeur de fragrance (310).

7. Dispositif selon la revendication 1, dans lequel le mécanisme de fragrance (124) comprend :
a) une première amenée de fragrance (314a) ;
b) une seconde amenée de fragrance (314b) ; et
c) un distributeur de fragrance (310) pouvant être raccordé de manière sélective à la première amenée (314a), à la seconde amenée (314b), ou ni à la première amenée ni à la seconde amenée.

8. Dispositif selon la revendication 7, dans lequel le distributeur de fragrance (310) peut également être raccordé de manière opérationnelle à la fois à la première amenée (314a) et à la seconde amenée (314b).

9. Dispositif selon la revendication 1, dans lequel le mécanisme de purification d'air (116) et le mécanisme de production de fragrance (124) sont formés dans une structure unique (1014) disposée dans le boîtier (1010).

10. Dispositif selon la revendication 9, dans lequel la structure unique (1014) comprend :
a) un cadre de milieu filtrant (1020) adapté pour y recevoir et retenir un milieu filtrant (1022) ; et
b) un mécanisme de distribution de fragrance (314, 316) disposé à l'intérieur du cadre (1020).

11. Dispositif selon la revendication 10, dans lequel le mécanisme de distribution de fragrance comporte au moins une zone capillaire (1024) qui peut se mettre en prise avec au moins une amenée de fragrance (314).

12. Dispositif selon la revendication 10, dans lequel le mécanisme de distribution de fragrance comprend :
a) un réservoir de fragrance (314) disposé à l'intérieur du cadre (1020) et adapté pour y maintenir une quantité de composition de fragrance ; et
b) une pluralité d'ouvertures de distribution (1027) en communication fluidique avec le réservoir (314) et avec une surface extérieure du cadre (1020) pour permettre de distribuer la composition de fragrance des ouvertures (1027) à l'intérieur du flux d'air.
